# DEMANDE DE BREVET EUROPEEN

(11) **EP 1 511 006 A2**
(43) Date de publication de la demande: **02.03.2005**
(21) Numéro de dépôt: 04292083.5
(22) Date de dépôt: 24.08.2004
(51) Int. Cl.: G10K 11/00

(54) **Procédé et dispositif avec un sonde ultrasonore**

(30) Priorité: 01.09.2003 FR 0310349
(71) Demandeur: CENTRE NATIONAL DE LA RECHERCHE SCIENTIFIQUE (CNRS), 75794 Paris Cedex 16 (FR)
(72) Inventeur: Fabre, Jean-Jacques, 59310 Auchy Les Orchies (FR); Lefebvre, Fabrice, 59144 Wargnies le Grand (FR); Waxin, Gérard, 59530 Ghissignies (FR)
(74) Mandataire: Burbaud, Eric

(57) **Abrégé**

Sonde de caractérisation ultrasonore comportant des transducteurs ultrasonores (T0, T'0, T1-T8) alignés selon une direction de propagation (X), disposés perpendiculairement à cette direction de propagation et adaptés pour vibrer dans leurs plans. Ces transducteurs sont reliés indépendamment les uns des autres à un même support (11) par des supports élastiques (12, 16) garantissant une force d'appui suffisante de chaque transducteur sur le milieu à caractériser.

## Description

La présente invention est relative aux sondes de caractérisation ultrasonore, aux dispositifs de caractérisation comprenant de telles sondes, et aux procédés de caractérisation utilisant de telles sondes.

Plus particulièrement, l'invention concerne une sonde de caractérisation ultrasonore destinée à mesurer au moins une caractéristique d'un milieu, cette sonde comportant des transducteurs ultrasonores adaptés pour être mis en contact avec le milieu à caractériser (le contact en question s'entend au sens d'un contact direct ou indirect permettant une transmission d'ondes ultrasonores entre chaque transducteur et le milieu à caractériser).

Une telle sonde de caractérisation, constituée par deux transducteurs séparés utilisables pour des mesures in vitro par transmission d'ondes de Lamb ultrasonores dans les os, a été décrite notamment par Lefebvre et al. (Development of a new ultrasonic technique for bone and biomaterials in vitro characterization, Wiley Interscience, 24 juin 2002). Les études rapportées dans ce document ont notamment montré l'intérêt d'utiliser une telle sonde ultrasonore pour caractériser les os, en particulier en vue de détecter l'ostéoporose chez des patients.

La sonde décrite dans ce document est bien adaptée pour des mesures effectuées en laboratoire, mais ne permet pas des mesures rapides et aisées hors du laboratoire. En particulier, les manipulations nécessitées par cette sonde sont relativement longues, puisqu'elles impliquent de déplacer l'un des transducteurs entre plusieurs positions en vue d'effectuer plusieurs mesures successives en ces positions.

Par ailleurs, le document JP-A-2001 305115 décrit une sonde de caractérisation ultrasonore destinée à mesurer au moins une caractéristique d'un milieu, cette sonde comportant des transducteurs ultrasonores adaptés pour être mis en contact avec le milieu à caractériser (le contact en question s'entend au sens d'un contact direct ou indirect permettant une transmission d'ondes ultrasonores entre chaque transducteur et le milieu à caractériser), comprenant un ensemble de transducteurs ultrasonores plats, alignés selon une direction de propagation (rectiligne ou non) et disposés chacun sensiblement perpendiculairement à ladite direction de propagation, chacun desdits transducteurs étant adapté pour transformer un signal électrique ondulatoire en vibrations radiales dans son plan et inversement, les transducteurs étant reliés indépendamment les uns des autres à un même support (directement ou indirectement), par des moyens élastiques adaptés pour garantir une force d'appui suffisante et contrôlée de chaque transducteur sur le milieu à caractériser.

La présente invention a notamment pour but de permettre un appui mieux contrôlé des transducteurs sur le milieu à caractériser.

A cet effet, selon l'invention, une sonde du genre en question est caractérisée en ce qu'elle comporte des moyens pour garantir une force d'appui prédéterminée des transducteurs sur le milieu à caractériser, ces moyens comprenant une poche flexible remplie de gaz sous pression interposée entre les transducteurs et le support.

La sonde selon l'invention est aisément manipulable car elle se présente comme un ensemble pré-assemblé, ne nécessitant pas de réglage de position des transducteurs les uns par rapport aux autres. L'un des transducteurs de la sonde peut être utilisé pour émettre des ondes ultrasonores dans le milieu à caractériser, tandis que les autres transducteurs peuvent être utilisés pour mesurer, en différentes positions, les caractéristiques des ondes ultrasonores transmises dans le milieu par le transducteur utilisé en émission.

Du fait de la disposition des transducteurs parallèlement les uns aux autres, ils peuvent être disposés le cas échéant très près les uns des autres, ce qui permet d'effectuer des mesures des ondes ultrasonores à faibles intervalles (notamment à des intervalles inférieurs à la longueur d'onde des ondes ultrasonores dans le milieu à caractériser) sans aucun déplacement des transducteurs utilisés en réception.

De plus, du fait du montage élastique indépendant des transducteurs de la sonde selon l'invention, ils ne s'influencent pas les uns les autres et on garantit une qualité de contact suffisante entre chacun desdits transducteurs et le milieu à caractériser, donc une bonne qualité de mesure.

Enfin, la poche flexible permet une égalisation des forces d'appui des transducteurs sur le milieu à caractériser.

Dans différents modes de réalisation de la sonde selon l'invention, on peut éventuellement avoir recours en outre à l'une et/ou à l'autre des dispositions suivantes :
- la sonde comporte en outre des moyens pour mesurer et régler la pression dans la poche flexible ;
- les transducteurs sont écartés deux à deux d'une distance inférieure à 5 mm ;
- chaque transducteur comporte une pastille piézoélectrique montée dans une plaquette formant guide d'ondes ultrasonores ;
- chaque plaquette formant guide d'ondes est réalisée en "plexiglas®" ;
- chaque transducteur est porté par une languette flexible ;
- chaque transducteur, vu dans la direction de propagation, présente une extrémité arrondie à l'opposé du support ;
- les languettes flexibles portant respectivement les transducteurs sont reliées entre elles par une bande de matière sensiblement parallèle à la direction de propagation, lesdites languettes flexibles s'étendant toutes dans une même direction à partir de ladite bande de matière, sensiblement perpendiculairement à ladite bande de matière ;
- chaque transducteur est porté par une contre-masse fixée à la languette flexible correspondante ;
- chaque contre-masse est fixée à la languette flexible correspondante par l'intermédiaire d'une couche d'isolant acoustique ;
- une poche flexible remplie de gaz sous pression est interposée entre les languettes flexibles et le support ;
- le support est solidaire d'une poignée.

Par ailleurs, l'invention a également pour objet un dispositif de caractérisation ultrasonore comportant :
- une sonde telle que définie ci-dessus,
- des moyens pour générer un signal électrique d'excitation adapté pour exciter l'un des transducteurs de la sonde avec ce signal électrique d'excitation afin d'émettre une onde ultrasonore dans le milieu à caractériser,
- et des moyens pour capter des signaux électriques générés par au moins certains des autres transducteurs de la sonde lors de la propagation de ladite onde ultrasonore dans le milieu à caractériser.

Avantageusement, le dispositif selon l'invention comporte aussi des moyens pour déterminer au moins un paramètre caractéristique du milieu à caractériser, à partir des signaux électriques captés.

Enfin, l'invention a encore pour objet un procédé de caractérisation pour déterminer au moins un paramètre caractéristique d'un milieu à caractériser, ce procédé comportant au moins les étapes suivantes :
(a) appliquer une sonde telle que définie ci-dessus contre le milieu à caractériser,
(b) générer un signal électrique d'excitation adapté pour exciter l'un des transducteurs de la sonde avec ce signal électrique d'excitation afin d'émettre une onde ultrasonore dans le milieu à caractériser,
(c) capter des signaux électriques générés par au moins certains des autres transducteurs de la sonde lors de la propagation de ladite onde ultrasonore dans le milieu à caractériser,
(d) déterminer au moins un paramètre caractéristique du milieu à caractériser, à partir des signaux électriques captés.

Dans différents modes de réalisation du procédé selon l'invention, on peut éventuellement avoir recours en outre à l'une et/ou à l'autre des dispositions suivantes :
- le milieu à caractériser comprend une partie du squelette d'un patient, et au cours de l'étape (a), on applique la sonde sur cette partie du squelette dudit patient ;
- au cours de l'étape (a), on applique les transducteurs directement contre la peau du patient recouvrant ladite partie du squelette ;
- au cours de l'étape (b), on génère une onde ultrasonore modulée de fréquence centrale comprise entre 100 kHz et 2 MHz ;
- au cours de l'étape (a), la sonde appliquée contre le milieu à caractériser présente, entre ses transducteurs, des écartements inférieurs à la longueur d'ondes, dans le milieu à caractériser, des ondes ultrasonores émises dans ce milieu par ladite sonde ;
- le matériau à caractériser comprend au moins un os et au cours de l'étape (d), on détermine au moins un paramètre de l'os, choisi parmi : la vitesse de propagation des ondes ultrasonores, l'atténuation des ondes ultrasonores, le module d'Young, l'épaisseur de l'os cortical, la densité et le coefficient de Poisson.

D'autres caractéristiques et avantages de l'invention apparaîtront au cours de la description suivante d'une de ses formes de réalisation, donnée à titre d'exemple non limitatif, en regard des dessins joints.

Sur les dessins :
- la figure 1 est une vue schématique illustrant un mode d'utilisation de la sonde de caractérisation ultrasonore selon une forme de réalisation de l'invention,
- la figure 2 est une vue schématique en perspective de la sonde de la figure 1,
- la figure 3 est une vue partielle en coupe de la sonde de la figure 2,
- et la figure 4 est un schéma bloc d'un dispositif de caractérisation ultrasonore incluant la sonde de la figure 2.

Sur les différentes figures, les mêmes références désignent des éléments identiques ou similaires.

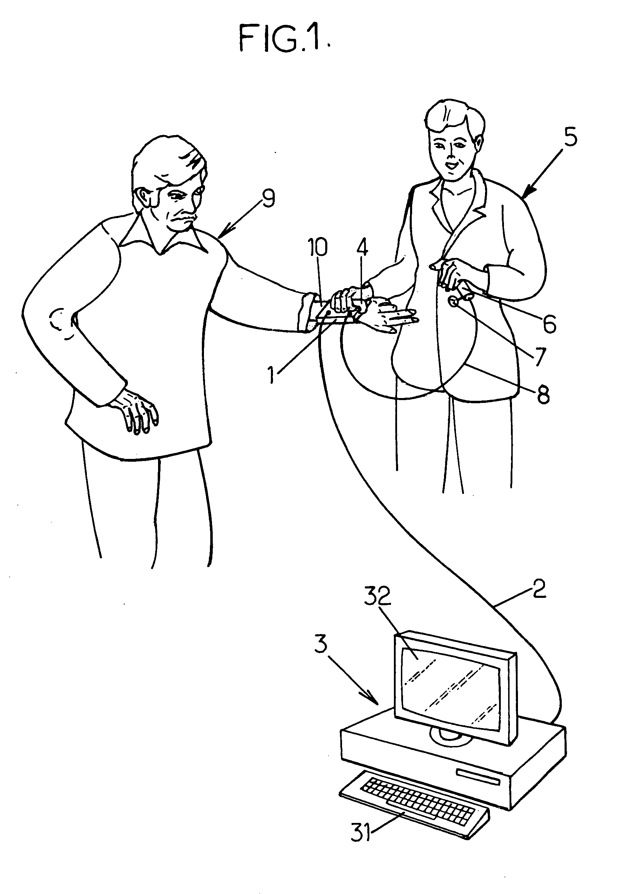

Comme représenté sur la figure 1, un dispositif de caractérisation ultrasonore selon l'invention peut comprendre une sonde de caractérisation ultrasonore 1, reliée par exemple par un câble 2 à un dispositif d'excitation et de traitement de signal 3.

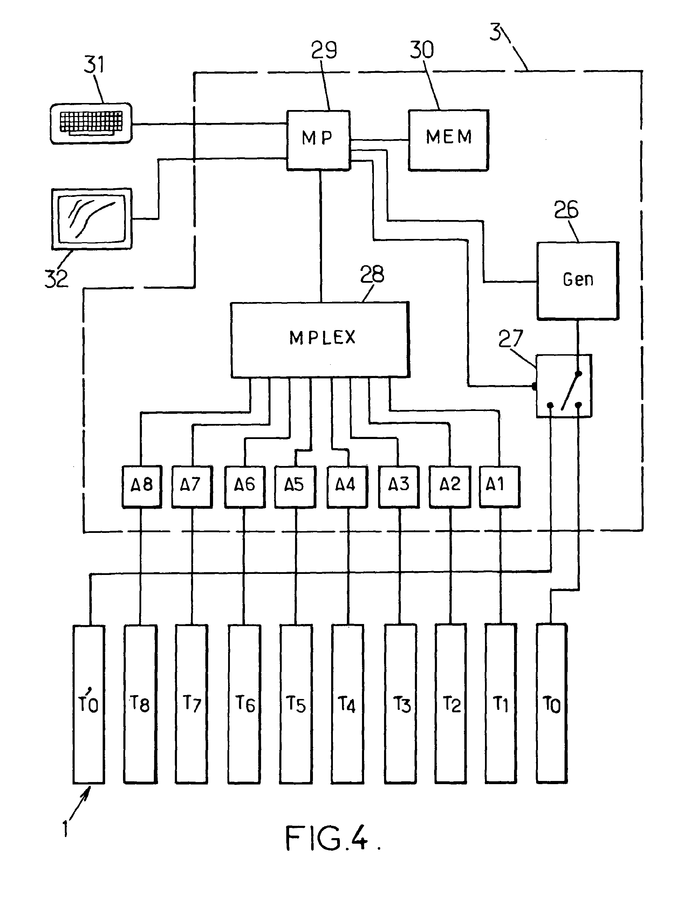

Ce dispositif d'excitation et de traitement de signal 3 peut être constitué notamment :
- soit par un appareil spécifique tel que par exemple un système de mesure modulable "NATIONAL INSTRUMENT" de type "PXI",
- soit par un micro-ordinateur pourvu de cartes électroniques appropriées permettant la génération et l'acquisition de signaux électriques dans les fréquences ultrasonores, comme expliqué plus en détails ci-après en regard de la figure 4.

La sonde 1 est de préférence portative et peut comporter à cet effet une poignée 4 destinée à être prise en main par un opérateur 5. Cet opérateur 5 peut avantageusement avoir également à sa disposition, une poire 6 ou autre appareil de gonflage pneumatique, de préférence associée à un manomètre 7, qui est reliée par un tuyau flexible 8 à la sonde 1 pour gonfler une poche souple appartenant à la sonde, comme il sera décrit plus en détails ci-après. La sonde 1 est utilisable pour caractériser :
- soit des matériaux divers dans des applications de contrôle non destructif, notamment en milieu industriel,
- soit des parties du squelette d'un patient 9 dans des applications médicales, notamment pour mesurer des paramètres des os permettant un diagnostic de l'ostéoporose par un praticien, ou pour toute autre application médicale.

Dans ce dernier cas, comme représenté sur la figure 1, la sonde 1 peut par exemple être appliquée directement sur la peau du patient 9, sans application préalable d'un gel ou autre milieu de couplage pour favoriser la transmission des ondes ultrasonores. Plus précisément, la sonde 1 peut par exemple être appliquée sur l'avant-bras 10 du patient, par exemple contre son cubitus, pour faire propager et capter des ondes ultrasonores, notamment des ondes de Lamb, dans la partie corticale de l'os (c'est à dire dans l'os dur externe).

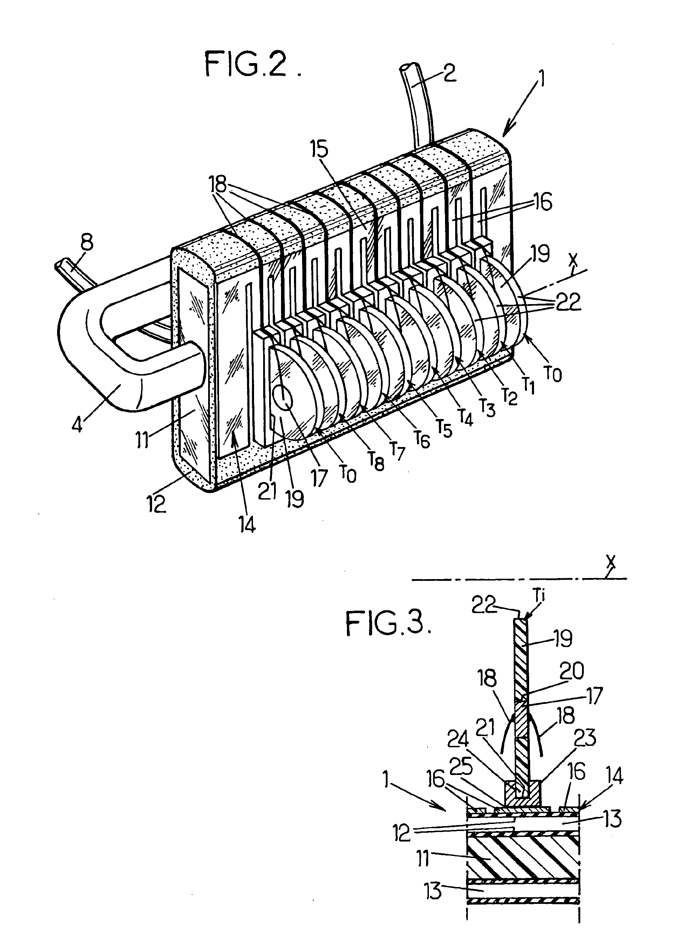

Comme représenté sur les figures 2 et 3, la sonde 1 peut comporter un support rigide 11, en matière plastique ou autre, qui peut être solidaire de la poignée 4 et autour duquel peut être disposée une poche souple 12 qui délimite un réservoir 13 rempli d'air à une pression relative prédéterminée (par exemple 40 ou 50 mmHg) par l'opérateur 5 au moyen de la poire de gonflage 6 susmentionnée. Cette poche souple 12, la poire de gonflage 6 et le manomètre 7 peuvent éventuellement être constitués par un tensiomètre médical dont la bande gonflable est enroulée autour du support 11 pour former la poche souple 12 susmentionnée.

Le support 11 et la poche souple 12 peuvent présenter par exemple une forme générale aplatie, avec deux faces principales orientées respectivement vers la poignée 4 et à l'opposé de ladite poignée. Sur la face principale opposée à la poignée 4 est disposée une fine plaque 15 de matière plastique ou autre matière flexible, qui recouvre la poche souple 12. Cette plaque 14 peut par exemple être collée sur la face externe de la poche souple 12, ou le cas échéant être fixée directement au support 11, la face interne de la plaque 14 étant néanmoins dans ce dernier cas au contact de la face externe de la poche souple.

La plaque 14 est découpée en forme de peigne, avec une bande de matière 15 s'étendant sensiblement dans la direction X de propagation des ondes ultrasonores et des languettes flexibles 16 s'étendant sensiblement perpendiculairement à la bande de matière 15, toutes dans la même direction à partir de cette bande de matière.

Vers l'extrémité libre de chaque languette flexible 16 est fixé un transducteur ultrasonore T0, T'0, T1, ... T8 de forme générale plane et perpendiculaire à la direction de propagation X, les transducteurs étant tous alignés selon la direction de propagation X (qui est ici rectiligne mais pourrait être éventuellement curviligne).

Dans l'exemple représenté sur les dessins, les transducteurs sont au nombre de 10 et comprennent :
- deux transducteurs d'extrémité T0, T'0 qui sont utilisés comme transducteurs d'émission pour générer des ondes ultrasonores lorsqu'ils reçoivent un signal électrique d'excitation dans la plage des fréquences ultrasonores,
- et huit transducteurs centraux T1-T8 qui sont utilisés comme transducteurs de réception pour capter les ondes ultrasonores transmises dans le milieu à caractériser par le transducteur d'émission T0 ou T'0.

Bien entendu, les transducteurs pourraient être en nombre différent de 10, avantageusement supérieur à 4, et on pourrait utiliser d'autres transducteurs que les transducteurs d'extrémité pour émettre les ondes ultrasonores dans le milieu à caractériser.

Dans l'exemple représenté sur les figures 2 et 3, chaque transducteur Ti comprend (voir figure 2) une pastille piézoélectrique 17 située dans le plan du transducteur et présentant par exemple une forme de disque de diamètre de l'ordre de par exemple 10 mm. Chaque pastille piézoélectrique peut présenter une fréquence propre comprise par exemple entre 100 kHz et 2 MHz, notamment de l'ordre de 220 kHz.

Les pastilles piézoélectriques 17 sont reliées par leurs deux faces respectivement à deux fils électriques 18 provenant du câble 2 pour :
- transmettre un signal électrique à la pastille piézoélectrique 17 du transducteur utilisé en émission, cette pastille piézoélectrique 17 étant alors adaptée pour vibrer radialement dans son plan,
- et capter un signal électrique généré par la pastille piézoélectrique 17 de chaque transducteur utilisé en réception, lorsque cette pastille est mise en vibration radialement dans son plan en recevant des vibrations ultrasonores qui se sont propagées dans le milieu à caractériser.

De plus, chaque transducteur Ti comporte également une plaquette 19 formant guide d'ondes ultrasonores, réalisée par exemple en "Plexiglas"®, qui comporte un évidemment central 20 dans lequel est emboîtée la pastille piézoélectrique dudit transducteur. Les plaquettes 19 sont acoustiquement adaptées aux pastilles piézoélectriques 17.

Chacune de ces plaquettes 19 formant guide d'ondes s'étend entre une base 21 liée à la languette flexible 16 correspondante, et une extrémité libre 22 adaptée à venir en appui contre le milieu à caractériser, par l'intermédiaire de la peau du patient 9 dans l'exemple considéré ici. Avantageusement, les extrémités libres 21, vues dans la direction de propagation X, présentent une forme arrondie permettant à la fois un contact sensiblement ponctuel et indolore avec la peau du patient 9. Dans l'exemple représenté sur les figures 2 et 3, les plaquettes 19 formant guides d'ondes présentent chacune une forme de disque, tronqué au niveau de sa base 21, de rayon par exemple de l'ordre de 35 mm.

La base 21 de chaque plaquette 19 peut par exemple être fixée à une contre-masse métallique 23, par exemple par emboîtement serré dans une rainure 24 de cette contre-masse, et ladite contre-masse 23 peut elle-même être fixée à l'extrémité libre de la languette flexible 16 correspondante par exemple par l'intermédiaire d'une couche d'isolant acoustique 25 telle qu'un ruban adhésif double face.

On notera que les dispositions précitées permettent de disposer les transducteurs à faibles intervalles les uns des autres, par exemple à des intervalles inférieurs à 5 mm et notamment inférieurs à la longueur d'onde des ondes ultrasonores dans le milieu à caractériser.

Comme représenté sur la figure 4, l'appareil 3 de traitement de signal peut comprendre par exemple :
- un générateur 26 (Gen) adapté pour générer par exemple des trains de 5 à 6 sinusoïdes d'ondes électriques, de fréquence centrale comprise par exemple entre 100 kHz et 2 MHz et notamment de l'ordre de 220 kHz (signal d'enveloppe gaussienne), ce générateur 26 pouvant par exemple être relié à un commutateur électrique ou électronique 27 adapté pour orienter les signaux électriques soit vers le transducteur T0, soit vers le transducteur T'0,
- huit amplificateurs à filtres passe-bande A1-A8 recevant respectivement les signaux générés par les transducteurs T1-T8,
- un multiplexeur 28 (MPLEX) relié aux sorties des amplificateurs A1-A8,
- et un microprocesseur 29 (MP) adapté pour acquérir les signaux en sortie du multiplexeur 28 et stocker ces signaux dans une mémoire 30 (MEM), le microprocesseur 29 étant relié, par des interfaces connues en soi, à divers périphériques tels qu'un clavier 31 et un écran 32, et ledit microprocesseur pouvant avantageusement commander le générateur de signaux 26 et le commutateur 27.

Le microprocesseur 29 peut éventuellement en outre être programmé pour traiter les signaux captés, afin de déterminer le ou les paramètres recherchés du matériau à caractériser, en temps réel ou de façon différée. En variante, les signaux captés peuvent également être traités sur un ordinateur distinct du dispositif de 3 susmentionné.

Le dispositif qui vient d'être décrit fonctionne comme suit, dans l'exemple considéré ici.
(a) L'opérateur 5 applique la sonde 1 directement sur la peau du patient 9 (sans gel ni autre milieu de couplage dans l'application considérée ici - un milieu de couplage tel que par exemple une couche d'élastomère peut être nécessaire dans des applications industrielles de contrôle non destructif), contre l'os à caractériser (figure 1). Au cours de cette étape, si l'os à caractériser est un os long (par exemple le cubitus), l'opérateur dispose la sonde 1 de façon que les transducteurs T0, T1-T8, T'0, viennent en appui sur l'os selon une ligne de contact disposée selon la direction longitudinale de l'os.
   Au cours de la même étape (a), l'opérateur 5 règle la pression dans le réservoir d'air 13 à une valeur prédéterminée, et appuie suffisamment sur la sonde 1 pour que toutes les languettes élastiques 16 qui portent les transducteurs fléchissent au moins légèrement, en comprimant la poche d'air 13 : on garantit ainsi une force d'appui prédéterminée de chaque transducteur sur l'os du patient, donc une qualité sensiblement identique de transmission des ondes ultrasonores pour tous les transducteurs. On notera que le réservoir pourrait être rempli d'un gaz autre que l'air, voire de liquide s'il est délimité par une poche élastique 12 (dans ce cas, on pourrait omettre la poire 6 et le tuyau 8). Eventuellement, le réservoir 12 pourrait être supprimé et remplacé par des vérins miniaturisés ou similaires permettant d'appliquer chaque transducteur sur le milieu à caractériser avec une force d'appui prédéterminée.
   Enfin, au cours de cette étape (a), l'opérateur 5 détermine également par quel transducteur T0 ou T'O l'onde ultrasonore doit être émise. L'opérateur peut par exemple indiquer son choix sur le clavier 31, après quoi le microprocesseur 29 actionne le commutateur 27 pour que les signaux sortant du générateur 26 alimentent le bon transducteur d'émission. On notera que, bien entendu, un des transducteurs T1-T8 pourrait également servir de transducteur d'émission. Par ailleurs, l'un des transducteurs T0, T'0 peut être supprimé, de même que le commutateur 27 : dans ce cas, l'opérateur oriente simplement la sonde dans le sens de propagation souhaité pour les ondes ultrasonores.
(b) Une fois la sonde en place, l'opérateur 5 déclenche (par exemple à l'aide du clavier 31) l'émission des ondes ultrasonores : le générateur 26 génère alors des signaux électriques d'excitation tels que décrits précédemment et les transmet par exemple au transducteur T0, qui génère des ondes ultrasonores de même fréquence que les signaux d'excitation et les transmet à l'os du patient 9.
(c) Ces ondes, du fait de leur fréquence relativement faible, peuvent se propager sur des distances suffisantes dans l'os cortical (c'est à dire au moins sur la longueur de la sonde, par exemple 5 à 15 cm). Au cours de cette propagation, les ondes ultrasonores sont captées par les transducteurs T1-T8, qui génèrent des signaux électriques représentatifs des ondes captées, lesquels signaux sont stockés dans la mémoire 30 du dispositif de traitement 3.
(d) A partir de ces signaux, on détermine en temps réel ou différé, au moins un paramètre caractéristique de l'os du patient, choisi parmi : la vitesse de propagation des ondes ultrasonores, l'atténuation des ondes ultrasonores, le module d'Young, l'épaisseur de l'os cortical, la densité et le coefficient de Poisson. A partir de tout ou partie de ces paramètres, un praticien peut déterminer par exemple si le patient souffre d'ostéoporose, par exemple en comparant les paramètres mesurés à des valeurs repères.

Parmi les paramètres susmentionnés, la vitesse et l'atténuation peuvent être déterminées directement à partir des signaux captés, de façon bien connue.

Les paramètres tels que le module d'Young et l'épaisseur de l'os cortical peuvent être déterminés de façon plus indirecte, par exemple par transformée de fourrier (FFT) spatiale des hodochrones des signaux captés puis en ajustant ces paramètres pour minimiser l'écart entre des courbes théoriques et les courbes réelles correspondantes (notamment par la méthode des moindres carrés), par exemple comme indiqué :
- dans l'article susmentionné de Lefebvre et al.,
- dans l'article de Ahite et al. intitulé "Nouvelle technique de caractérisation de l'os par les ultrasons : étude préliminaire in vitro" (Innov. Techn. Biol. Med., Vol 19, n°4, 1998),
- dans-l'article de Lefebvre et al. intitulé "Bone characterization using Lamb's waves" (Proceeding of 6^{th} C.F.A. Lille 8-11 avril 2002),
- ou dans la thèse de D. Ahite soutenue le 9 juin 2000 à l'université de Valenciennes et du Hainaut Cambrésis ("Contribution à la mise au point d'un dispositif de caractérisation ultrasonore du tissu osseux cortical par ondes de lamb").

La densité et le coefficient de Poisson peuvent être déterminés de façon similaire.

## Revendications

1. Sonde de caractérisation ultrasonore destinée à mesurer au moins une caractéristique d'un milieu, cette sonde comportant des transducteurs ultrasonores (T0, T'0, T1-T8) adaptés pour être mis en contact avec le milieu à caractériser, comprenant un ensemble de transducteurs ultrasonores (T0, T'0, T1-T8) plats, alignés selon une direction de propagation (X) et disposés chacun sensiblement perpendiculairement à ladite direction de propagation, chacun desdits transducteurs étant adapté pour transformer un signal électrique ondulatoire en vibrations radiales dans son plan et inversement, les transducteurs (T0, T'0, T1-T8) étant reliés indépendamment les uns des autres à un même support (11), par des moyens élastiques (13, 16) adaptés pour garantir une force d'appui suffisante et contrôlée de chaque transducteur (T0, T'0, T1-T8) sur le milieu à caractériser, **caractérisée en ce qu'**elle comporte des moyens (12, 13) pour garantir une force d'appui prédéterminée des transducteurs sur le milieu à caractériser, ces moyens comprenant une poche flexible (12) remplie de gaz sous pression interposée entre les transducteurs (T0, T'0, T1-T8) et le support (11).

2. Sonde selon la revendication 1, comportant en outre des moyens (6) pour régler la pression dans la poche flexible (12).

3. Sonde selon l'une quelconque des revendications précédentes, dans laquelle les transducteurs (T0, T'0, T1-T8) sont écartés deux à deux d'une distance inférieure à 5 mm.

4. Sonde selon l'une quelconque des revendications précédentes, dans laquelle chaque transducteur (T0, T'0, T1-T8) comporte une pastille piézoélectrique (17) montée dans une plaquette (19) formant guide d'ondes ultrasonores.

5. Sonde selon la revendication 4, dans laquelle chaque plaquette (19) formant guide d'ondes est réalisée en "plexiglas".

6. Sonde selon l'une quelconque des revendications précédentes, dans laquelle transducteur (T0, T'0, T1-T8), vu dans la direction de propagation (X), présente une extrémité arrondie (21) à l'opposé du support (11).

7. Sonde selon l'une quelconque des revendications précédentes, dans laquelle chaque transducteur (T0, T'0, T1-T8) est porté par une languette flexible (16).

8. Sonde selon la revendication 7, dans laquelle les languettes flexibles (16) portant respectivement les transducteurs (T0, T'0, T1-T8) sont reliées entre elles par une bande de matière (15) sensiblement parallèle à la direction de propagation (X), lesdites languettes flexibles (16) s'étendant toutes dans une même direction à partir de ladite bande de matière (15), sensiblement perpendiculairement à ladite bande de matière.

9. Sonde selon la revendication 7 ou la revendication 8, dans laquelle chaque transducteur (T0, T'0, T1-T8) est porté par une contre-masse (23) fixée à la languette flexible (16) correspondante.

10. Sonde selon la revendication 9, dans laquelle chaque contre-masse (23) est fixée à la languette flexible (16) correspondante par l'intermédiaire d'une couche d'isolant acoustique (25).

11. Sonde selon l'une quelconque des revendications 7 à 10, dans laquelle une poche flexible (12) remplie de gaz sous pression est interposée entre les languettes flexibles (16) et le support (11).

12. Sonde selon l'une quelconque des revendications précédentes, dans laquelle le support (11) est solidaire d'une poignée (4).

13. Sonde selon l'une quelconque des revendications précédentes, comportant en outre des moyens de réglage (6, 7) pour régler la force d'appui des transducteurs sur le milieu à **caractériser**.

14. Dispositif de caractérisation ultrasonore comportant :
- une sonde (1) selon l'une quelconque des revendications précédentes,
- des moyens (26) pour générer un signal électrique d'excitation adapté pour exciter l'un des transducteurs (T0, T'0) de la sonde avec ce signal électrique d'excitation afin d'émettre une onde ultrasonore dans le milieu à **caractériser**,
- et des moyens (30) pour capter des signaux électriques générés par au moins certains des autres transducteurs (T1-T8) de la sonde lors de la propagation de ladite onde ultrasonore dans le milieu à **caractériser**.

15. Dispositif selon la revendication 14, comportant en outre des moyens (30) pour déterminer au moins un paramètre caractéristique du milieu à **caractériser**, à partir des signaux électriques captés.

16. Procédé de caractérisation pour déterminer au moins un paramètre caractéristique d'un milieu à **caractériser**, ce procédé comportant au moins les étapes suivantes :
(a) appliquer une sonde (1) selon l'une quelconque des revendications 1 à 13 contre le milieu à **caractériser**,
(b) générer un signal électrique d'excitation adapté pour exciter l'un des transducteurs de la sonde avec ce signal électrique d'excitation afin d'émettre une onde ultrasonore dans le milieu à **caractériser**,
(c) capter des signaux électriques générés par au moins certains des autres transducteurs de la sonde lors de la propagation de ladite onde ultrasonore dans le milieu à **caractériser**,
(d) déterminer au moins un paramètre caractéristique du milieu à **caractériser**, à partir des signaux électriques captés.

17. Procédé selon la revendication 16, dans lequel le milieu à **caractériser** comprend une partie du squelette d'un patient, et au cours de l'étape (a), on applique la sonde sur cette partie du squelette dudit patient.

18. Procédé selon la revendication 17, dans lequel au cours de l'étape (a), on applique les transducteurs directement contre la peau du patient recouvrant ladite partie du squelette.

19. Procédé selon l'une quelconque des revendications 16 à 18, dans lequel au cours de l'étape (b), on génère une onde ultrasonore modulée de fréquence centrale comprise entre 100 kHz et 2 MHz.

20. Procédé selon l'une quelconque des revendications 16 à 19, dans lequel au cours de l'étape (a), la sonde appliquée contre le milieu à **caractériser** présente, entre ses transducteurs (T0, T'0, T1-T8), des écartements inférieurs à la longueur d'ondes, dans le milieu à **caractériser**, des ondes ultrasonores émises dans ce milieu par ladite sonde.

21. Procédé selon l'une quelconque des revendications 16 à 20, dans lequel le matériau à **caractériser** comprend au moins un os et au cours de l'étape (d), on détermine au moins un paramètre de l'os, choisi parmi : la vitesse de propagation des ondes ultrasonores, l'atténuation des ondes ultrasonores, le module d'Young, l'épaisseur de l'os cortical, la densité et le coefficient de Poisson.
